# EUROPEAN PATENT SPECIFICATION

(11) **EP 2 121 094 B1**
(45) Date of publication and mention of the grant of the patent: **25.04.2018**
(21) Application number: 07727061.9
(22) Date of filing: 19.03.2007
(51) Int. Cl.: A61M 16/01, A61M 16/20, G06F 3/01, A61M 16/00

(54) **DEVICE FOR MANUAL INPUT AND HAPTIC OUTPUT OF PATIENT CRITICAL OPERATING PARAMETERS IN A BREATHING APPARATUS**
SYSTEM FÜR MANUELLEN INPUT UND HAPTISCHEN OUTPUT VON KRITISCHEN BEDIENUNGSPARAMETERN EINES PATIENTEN IN EINEM ATEMGERÄT
DISPOSITIF PERMETTANT L'ENTRÉE MANUELLE ET LA SORTIE HAPTIQUE DES PARAMÈTRES DE FONCTIONNEMENT CRITIQUES POUR LE PATIENT DANS UN APPAREIL RESPIRATEUR

(43) Date of publication of application: 25.11.2009
(73) Proprietor: Maquet Critical Care AB, 171 06 Solna (SE)
(72) Inventor: TORNESEL, Carl-Magnus, S-121 37 Johanneshov (SE); MALM, Jan, S-175 69 Järfälla (SE)
(74) Representative: Zacco Sweden AB
(86) International application number: PCT/EP2007/052583
(87) International publication number: WO 2008/113410

(56) References cited:
- EP-A- 1 287 843
- EP-A- 1 421 966
- US-A- 5 950 623
- US-A1- 2006 038 781
- US-A1- 2006 255 683
- US-B1- 7 038 667

## Description

### Field of the Invention

This invention pertains in general to the field of manual input devices in breathing apparatuses devices for adjusting operating parameters thereof. More particularly the invention relates to a device for providing manual input of patient critical operating parameters to the breathing apparatus. In an embodiment, the invention relates to providing manual input for operating an adjustable pressure-limiting valve of an anesthesia patient breathing circuit in a respiratory anesthesia delivery system.

### Background of the Invention

Anesthesia patient breathing circuits are utilized to convey gasses containing an anesthetic vapor to a patient to carry out the sedation of the patient. An anesthesia machine provides a mixture of gases and vaporized anesthetic agents. This mixture is conveyed to the patient via the anesthesia patient breathing circuit. In order to limit the maximum pressure during manual ventilation, an adjustable pressure limiting (APL) valve is provided in the anesthesia patient breathing circuit. The APL valve is a pressure relief valve that vents the anesthesia patient breathing circuit when the pressure within the circuit reaches a predetermined level, such that the patient is not subjected to an excessive pressure. The APL valve is adjustable by the user so that differing maximum pressures are allowed in the patient breathing system during an operation and can be determined by the user. The opening pressure of the APL valve is a patient critical operating parameter.

During manual ventilation the operator, usually an anesthesiologist, is, as a rule, placed close to the patient, and with one hand operates a manual ventilation bladder. The other hand of the operator controls for instance a facemask on the patient and adjusts parameters on the anesthesia machine, such as the maximum pressure in the anesthesia patient breathing circuit by means of the APL valve. This usually occurs at the induction of anesthesia, prior to surgery, during the course of administering a sedative agent intravenously and the commencing of machine assisted ventilation by means of the anesthesia machine. Furthermore, this occurs at the conclusion of anesthesia, after surgery, when awakening the patient from sedation and returning the patient to spontaneous breathing. During these critical phases, the anesthesiologist is very much occupied of handling the manual ventilation bladder and the patient. A number of physiological parameters, such as concentration of inhaled and exhaled gases, blood oxygenation, ECG, EEG, etc. are observed by the anesthesiologist simultaneously with manually ventilating the patient via the ventilation bladder. Hence, during these intense phases, the anesthesiologist has a very limited possibility of visually controlling the anesthesia machine.

US 5,950,623 discloses an adjustable pressure-limiting (APL) valve having a non-linear biasing means. The APL valve has a movable valve member that can be moved to an open position by a predetermined pressure and a closed position on a valve seat. By rotating a control knob, the user adjusts a bias acting against the movable valve member towards the closed position to set the pressure at which the valve opens. The control knob is mechanically coupled to the movable valve member. More precisely, a spring acting against the movable valve member is compressed or decompressed by rotating the control knob. By mechanically providing a non-linear relationship between the rotational movement of the control knob and compression or decompression of the spring, a non-linear biasing of the APL valve is achieved.

However, the APL valve disclosed in US 5,950,623, like all directly mechanically operated APL valves, has a number of drawbacks. Mechanical solutions are very costly when a repeatability with close tolerances is required, as in the present case. Furthermore, the mechanical construction of the valve operating unit is subject to wear and tear, which on the one hand limits the life of the operating unit, and which on the other hand leads to an undesired variation of the adjustment mechanism over time. Moreover, a tactile feedback, which is desired so that the user does not have to look at the control knob when operating the latter, is limited to certain predefined rotational angles. Further, the tactile feedback that may be provided by such mechanical solutions is purely passive, e.g. the force overcoming the spring load in both directions. Moreover, the tactile feedback that is provided is often limited to a single type of tactile feedback, as in the case of the valve disclosed in US 5,950,623, where two regions with different thread pitches control the compression of a spring acting against the valve member. In addition, the patient pressure directly acts against and influences the spring load, which is undesired as the tactile feedback from the control knob is directly influenced thereby. Finally, APL valves of the type disclosed in US 5,950,623 need to be sterilized between patients, e.g. by autoclaving the control knob and valve mechanism. This contributes to an accelerated wear of the APL valve mechanism.

US 6,834,647 or corresponding EP 1287 843 disclose a remote control for a respiratory ventilator, allowing an operator to move with respect to the ventilator and a patient. However, there is no direct feedback of the magnitude of a parameter set by the operator while setting it, only a non-haptic feedback is disclosed. These documents are described in more detail further below.

An electronic solution for controlling an APL valve is disclosed in EP-A1-1421966 of the same applicant as the present application. More precisely, an anesthetic device is disclosed comprising a remote control for wireless transmission of commands to a user interface, which carries out commands from the remote control, only when the anesthetic device is set for manual ventilation. In this connection, the remote control includes controls for machine parameters, such as permitted over-pressure level via an electronically controlled APL valve. In an embodiment, the controls are realized as a wheel, analogous to a computer mouse wheel, but with a definite position related to a predetermined setting of the machine parameters. These machine parameters may be permanently programmed for the device, programmable for every patient or comprise values that are programmed by the operator for use when manual ventilation is switched in. With the last mentioned alternative the wheel may automatically take up a distinct position as manual ventilation is switched in. This may be achieved using a control signal from the user interface to the remote control and a small drive motor for the wheel responsive to this signal.

Even though the solution provided in EP-A1-1421966 addresses the drawbacks of mechanical APL valve control units, it does not provide the user with a tactile feedback in which pressure region the APL is adjusted by scrolling the wheel. The small drive motor may only establish a defined starting point, e.g. at a low pressure limit, and turning the wheel in one or the other direction lowers or raises the opening pressure of the APL valve. However, once the operator has started scrolling the wheel back and forth, a visual feedback with a control display on the anesthesia machine is needed to establish a currently adjusted pressure limit of the APL valve. A tactile feedback of the currently adjusted pressure limit regulated by the APL valve is not provided.

Thus, there is a need for an improved method and device for adjusting patient critical operating parameters. A patient critical operating parameter is for instance the maximum pressure limit in an anesthesia patient breathing circuit, which may be adjustable e.g. by means of an APL valve.

Hence, an improved or alternative method and device for at least one adjusting patient critical operating parameter, such as providing a control value for the adjustable pressure limit of an APL valve of an anesthesia patient breathing circuit, would be advantageous and in particular, an operating method and device allowing for increased flexibility, cost-effectiveness, and/or user friendliness would be advantageous.

### Summary of the Invention

Accordingly, embodiments of the present invention preferably seek to mitigate, alleviate or eliminate one or more deficiencies, disadvantages or issues in the art, such as the above-identified, singly or in any combination by providing a device according to the appended patent claims.

According to a first aspect of the invention, a breathing apparatus is provided comprising a manual input-output device, wherein said manual input-output device is devised for providing a manual input for adjusting at least one patient critical operating parameter of said breathing apparatus, wherein said manual input-output device is programmable for a haptic feedback upon adjustment of said at least one patient critical operating parameter by said manual input-output device, and comprises an operating member that is adapted to provide said manual input of said at least one patient critical operating parameter, and that is adapted to provide a manual output, a detecting unit that is configured to detect a movement of said operating member upon said manual input for adjusting at least one patient critical operating parameter of said breathing apparatus, wherein a haptic feedback unit that is configured to apply said manual output as a mechanical output to said operating member depending on said movement detected. Further embodiments of the invention are defined in the attached dependent claims.

Some embodiments of the invention provide for a programmable contactless mode of operation of an APL valve operating unit, such as a rotating operating knob.

Some embodiments of the invention also provide for a non-mechanical wearless operation of an APL valve operating unit.

Some embodiments of the invention provide for a tactile feedback of a patient critical operating parameter, such as an opening pressure of an APL valve, at a manual input device therefore, which is programmable with regard to position, activation force, vigor, intensity, magnitude, direction of operation, etc.

Some embodiments of the invention provide for operation of an APL valve operating unit that is operable immediately, e.g. at activation thereof, without a repositioning of the APL valve operating unit to a defined initial position.

Some embodiments of the invention provide for a distinct haptile feedback in which pressure range the APL valve operating unit is operated at a defined time of operation.

Some embodiments provide for improved patient safety when adjusting patient critical operating parameters of a breathing apparatus.

It should be emphasized that the term "comprises/comprising" when used in this specification is taken to specify the presence of stated features, integers, steps or components but does not preclude the presence or addition of one or more other features, integers, steps, components or groups thereof.

### Brief Description of the Drawings

These and other aspects, features and advantages of which embodiments of the invention are capable of will be apparent and elucidated from the following description of embodiments of the present invention, reference being made to the accompanying drawings, in which
Fig. 1 is a schematic illustration of an anesthetizing system having an anesthesia patient breathing circuit and an adjustable pressure limiting (APL) valve;
Fig. 2 is a schematic illustration of an embodiment of a manual input device for manually adjusting a patient critical operating parameter in the form of an opening pressure level of an electronic APL valve;
Fig. 3 is a schematic illustration of different operational modes in different rotational ranges of the manual input device;
Fig. 4A is a graph illustrating rotation of the manual input device in a first direction and corresponding haptic feedback;
Fig. 4B is a graph illustrating rotation of the manual input device in a second direction, opposite the first direction, and corresponding haptic feedback; and
Fig. 5 is a schematic illustration of a further embodiment of manual input device for manually adjusting an opening pressure level of an electronic APL valve, as well as other operating parameters of an anesthesia delivery system.

### Description of embodiments

Specific embodiments of the invention will now be described with reference to the accompanying drawings. This invention may, however, be embodied in many different forms and should not be construed as limited to the embodiments set forth herein; rather, these embodiments are provided so that this disclosure will be thorough and complete, and will fully convey the scope of the invention to those skilled in the art. The terminology used in the detailed description of the embodiments illustrated in the accompanying drawings is not intended to be limiting of the invention. In the drawings, like numbers refer to like elements.

The following description focuses on an embodiment of the present invention applicable to a manual input device for an anesthesia machine, and according to an embodiment to a manual input device providing a haptic feedback for adjusting an APL valve in an anesthesia patient breathing circuit of the anesthesia machine.

Haptics or Force Feedback refers to the technical field where the sense of touch is introduced into a human-machine interface. Conventionally, user interface devices of breathing apparatuses are input-only. They are tracking physical manipulations for an input but providing no physical feedback representing the results of such manipulations. Haptic input devices according to embodiments allow users to interact directly with a breathing apparatus. The user receives an interactive feel like of its selections, rather than just interacting with an input device that always feels the same. Furthermore, the haptic feedback is programmable and may be adapted to different patient or operating situations of the breathing apparatus. Providing an input device with haptic feedback virtually renders the input device an output device at the time of manipulation.

A controllable haptic feedback of an input device refers to a haptic feedback that is programmable or adjustable, for instance in dependence of a current operating situation, patient situation, or user input.

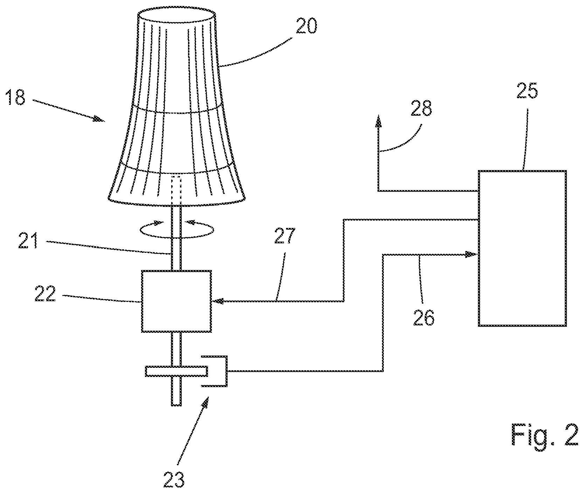

However, it will be appreciated that the invention is not limited to the specific application of providing haptic feedback for adjusting the APL valve in the anesthesia patient breathing circuit, but may in other embodiments be applicable to many different breathing apparatuses, or may in certain embodiments be applied to many other parameters related to the breathing apparatus, including for example manual input of patient critical operating parameters, such as positive end-expiratory pressure (PEEP), upper inspiratory pressure limit, pop off pressure, freshgas flow, tidal volume, inspiratory volume, gas concentrations, such as oxygen (O₂) concentration, nitrous oxide (N₂O) concentration, nitric oxide (NO) concentration, concentrations of anesthetic agents, e.g. delivered by means of vaporizers, etc. According to embodiments, these parameters may have associated individual haptic feedback profiles therewith, which are programmed into a manual input device of the breathing apparatus, such as a rotary knob of a ventilator or anesthesia machine. A manual input device in form of a rotary knob for adjusting parameters of a breathing apparatus without feedback is for instance disclosed in US 5,678,539 and in particular knob 21 of Fig.2 and col. 4, l. 25-55 of US 5,678,539. The same manual input device may be operated without haptic feedback for other parameters, which for instance are non-patient critical, and which are adjustable by means of the manual input device, such as input of a breathing mode, a patient name, etc. By using the invention for one or more of these parameters, patient safety may advantageously be increased by providing a haptic feedback apart from the visual feedback provided by a user interface, such as a display, bar readings etc.

US 6,834,647 or corresponding EP 1287 843 disclose a remote control for a respiratory ventilator, allowing an operator to move with respect to the ventilator and a patient. A parameter signal indicative of a specific patient or ventilator related respiratory or physiological parameters (col. 8, 1.36-41 in US 6,834,647), such as airway pressure, tidal volume, pulse rate, blood pressure, arterial blood oxygenation saturation, is provided from a sensor associated with the patient or ventilator (col. 3, 1. 20-21 in US 6,834,647). Data related to a selected parameter of this plurality of available sensed respiratory or physiological parameters is shown on a monitor, or provided to the remote control via a terminal (32, col. 3, 1. 51-58 and col. 4, l. 22-26 in US 6,834,647) for providing a tactile feedback of that specific selected respiratory or physiological parameter.

It is pointed out that the data sent to the remote control is always based on a sensor output related to a reading of a current parameter made by the sensor. Such sensors are for instance gas pressure sensors, gas flow sensors, blood pressure sensors, pulse oximeters, etc. A switch (60) is provided to select which of the plurality of parameters is to be sent to the remote control (col. 5, l. 35-40 in US 6,834,647).

The remote control of US 6,834,647 comprises a member for being received in the hand of the operator. The member comprises a movable trigger for being grippingly engaged by the fingers of the operator. A force applying element is coupled to the trigger for applying a force to the fingers of the operator responsive to the parameter signal received from the terminal. A tactile sensation of the actual value of the parameter is thus provided to the user. Movement of the trigger may provide a separate control signal to remotely control the ventilator (col. 4, 1.1-16 in US 6,834,647).

However, the remote control system of US 6,834,647 does not provide a direct tactile feedback based on the control signal (see Fig. 2, signal 88 is one-way, and col. 9, 1. 8-16 in US 6,834,647) given by the operator. The feedback is always derived from the sensor output, as mentioned above. Moreover, according to embodiments described in US 6,834,647 or EP 128 78 43, generally a different physiological or ventilatory parameter is fed back to the remote control trigger for tactile feedback than the physiological or ventilatory parameter that is controlled by the trigger. A preferred embodiment of US 6,834,647 or EP 128 78 43 is the simulation of a breathing bag, where the tidal volume is controlled by the manual action of the trigger and the patient pressure provided from an airway pressure (84 in Fig. 2) determines the tactile feedback (e.g. col. 6, 1.42-57 in US 6,834,647).

It is not explicitly mentioned in US 6,834,647 or EP 128 78 43, and it cannot be read onto the different embodiments mentioned therein, that the same physiological or ventilatory parameter may both be adjusted by the trigger of the remote control and a feedback be provided from the same physiological or ventilatory parameter to the trigger for tactile feedback. Even if this would be made, having the presently described invention in mind and against the teaching of US 6,834,647 or EP 128 78 43, the inherent transition time and time delay due to the intermediate sensor providing the feedback signal, would make the system of unusable in practice as patient safety could be at risk. For instance, when a patient critical operating parameter was adjusted relatively quickly, i.e. significantly faster than the effect of the adjustment is fed back, over a certain range via the control signal, patient safety may not be guaranteed. Patient critical operating parameters are for instance the opening pressure of an APL valve, maximum inspiratory patient pressure, etc. For instance if the allowed value for the maximum inspiratory patient pressure is quickly increased over a large range via the control signal, e.g. by pressing the trigger of the remote control quickly all the way in at an existing low inspiratory patient pressure, a control signal corresponding to a dangerously high inspiratory patient pressure may be set without giving the user a feedback on this adjustment. The setting of this dangerously high inspiratory patient pressure would not be hindered by a high resistance of the tactile feedback, because the signal from a corresponding pressure sensor at that moment indicates a low value, implying a low resistance in the trigger to the hand of the operator. Only when an increasing inspiratory patient pressure subsequently would increase, the corresponding feedback signal thus also would increase resistance in the trigger grip. However, as the trigger may already be pressed in all the way, the increasing resistance would not at all be given as a tactile feedback to the operator. The operator would thus not be aware of the dangerously high inspiratory patient pressure. Similarly, critical situations may arise when such patient critical operating parameters were adjusted by means of the remote control during expiration, e.g. a very high tidal volume may be selected during expiration, which would result in an extremely high inspiratory flow during the subsequent inspiratory phase, without the operator being aware of this adjustment from a tactile feedback. Thus, there is no direct feedback of the magnitude of a parameter set by the operator while setting it, only a non-haptic feedback is disclosed.

In contrast to this, patient critical operating parameters are adjustable according to embodiments of the present invention without risking patient safety.

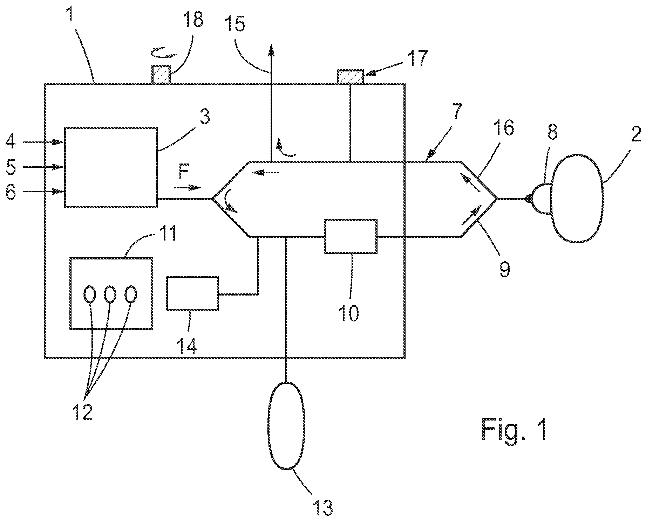

Now turning to the embodiments, in Fig.1, an anesthetizing system 1 is schematically shown. The anesthetic device 1 is for instance a anesthesia machine, and is connected to a patient 2 to be anaesthetized. A fresh gas flow F is provided from a fresh gas unit 3 comprising a first gas connection 4 for nitrous oxide, a second gas connection 5 for oxygen, and a third gas connection 6 for compressed air. The fresh gas unit 3 provides blending of a chosen mixture of laughing gas and oxygen or a chosen mixture of air and oxygen. The blend is provided to the patient breathing circuit 7 as a fresh gas flow F. Gas flow directions are indicated by the arrows in Fig. 1. Other arrangements for the preparation of fresh gas are known and are not further elucidated herein. Initially the fresh gas is conveyed via the patient breathing circuit 7 through an inspiratory tube 9 to a facemask 8, which is placed over the mouth and nose of the patient 2. The fresh gas is possibly supplied with an anesthetic agent. Alternatively, a vaporizer 10 provides for an anesthetic agent in the patient breathing circuit 7. The patient breathing circuit 7 may be operated in a variety of different operating modes, for example as an open system without re-breathing; a half-open system such as Bain and Mapelson with partial re-breathing; or a closed system with substantial re-breathing. Various designs of the facemask 8 are known and even tracheal or tracheotomy tubes may be employed instead of a facemask 8. Expiratory gases are conveyed via an expiratory tube 16 through the patient circuit 7. Expiratory gases are either discarded from the anesthesia machine 1 through an exhaust 15, e.g. via a "pop off" valve to an evacuation system, in an open system without re-breathing, or at least partly returned to the patient in a half-open or closed system. A "pop off" valve is referred to as an overpressure relief valve that controls the maximum pressure in the patient breathing circuit during mechanical ventilation.

The patent breathing circuit 7 may include an absorber (not shown) to rid the recirculating gases within the patient circuit of CO₂ to prevent a CO₂ build-up, various check valves, which insure that the flow of gas within the patient circuit is in the proper direction, and also a pressure relieving valve that vents the patient breathing circuit when the pressure within the circuit reaches a predetermined point so that the patient is not subjected to any excessive pressure. The pressure relief valve is referred to as an adjustable pressure limiting (APL) valve 17 and is adjustable by the user so that differing maximum pressures are allowed in the patient breathing circuit 7 during an operation and can be determined by the user. APL valve 17 is coupled to an evacuation system for managing gases exhaust from the breathing circuit 7 through the APL valve 17.

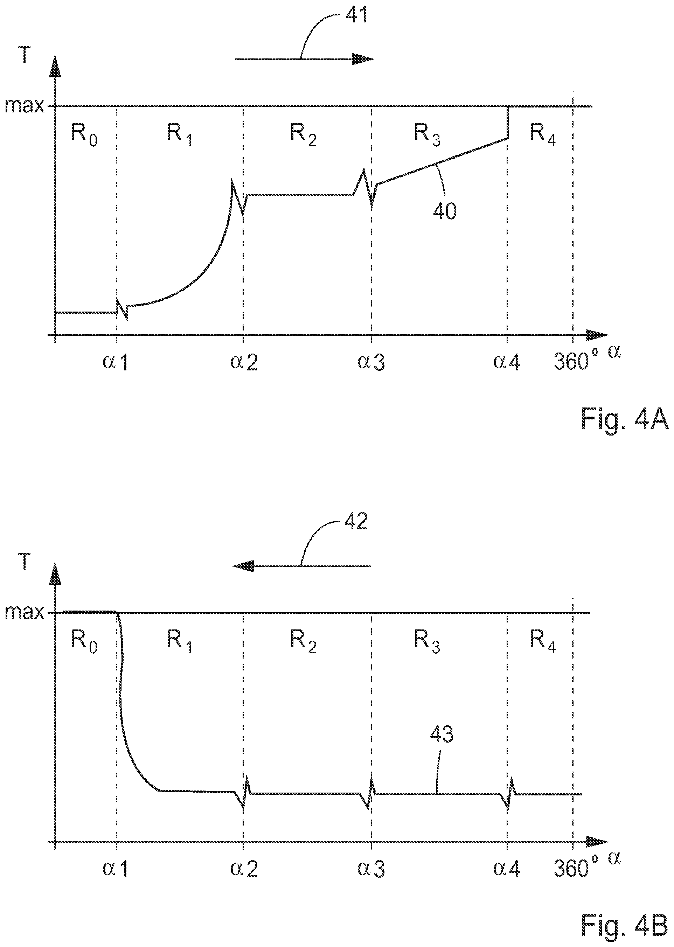

In a traditional mechanical breathing device often both an APL valve and a pop off valve are present. However, in an electronic system a single valve may provide the functions of both an APL valve and a pop off valve, as the input device no longer is mechanically coupled to the electronically regulated maximum pressure controlling valve. For instance the patent application PCT/EP2006/070068 of the same applicant as the present published as WO2007/071756 discloses such an integrated valve. In particular, Fig. 7 and the corresponding description of PCT/EP2006/070068 describes a ventilation system configured for manual and mechanical ventilation having a pop-off valve and an APL valve. Furthermore, Fig. 1 and Fig. 4 and the corresponding description of PCT/EP2006/070068 describes a ventilation system having an electronically controlled APL valve.

Embodiments of the present invention may be implemented with other ventilation systems than described herein, such as ventilation systems as described in PCT/EP2006/070068.

Returning to Fig. 1, an input for adjustment of the opening pressure of the APL valve 17 is made by means of a manual input-output device 18. The manual input-output device 18 is remote to the APL valve. Further, the manual input-output device 18 is programmable for a haptic feedback and comprises an operating member 20 for providing the manual input and output, a detecting unit that detects a movement of the operating member 20, and a haptic feedback unit 22 that applies a mechanical output to the operating member 20 depending on the movement thereof that is detected. The manual input-output device 18 will be described below in more detail with reference to Figs. 2, 3, 4A and 4B.

A user may set operating modes for the anesthetic device 1 as well as parametric values by means of a user interface 11. The user interface 11 has, in the present example, some form of input units 12. The user interface 11 may be based on physical input units, software based input units, such as interactive screens, or a combination thereof.

The anesthetic device 1 is able to be operated in both manual and mechanical ventilation modes and is therefore provided with a manual ventilation bladder 13 and a mechanical ventilator 14. The connection of these to the patient circuit 7 may be made in many different known ways and does not need to be further described in detail.

During mechanical ventilation, a continuous freshgas flow is given via a bag-in-bottle device or by direct ventilation. Non-return valves control the direction of flow in the breathing circuit and excess gas is vented through an exhaust via the pop-off valve.

During manual ventilation, a freshgas flow is supplied to the patient, breathing spontaneously. The patient's respiration may be supported by means of the manual ventilation bladder. Excess gas is vented through an exhaust via the APL valve 17. An opening pressure, determining the amount of excess gas being vented, is determined by the APL valve 17.

During manual ventilation of the patient 2, the operator, as a rule, needs to closely monitor the patient 2 as well as the manual ventilation bladder 13, as already was described above.

The manual input device 18 is mechanically completely disconnected from the electronic APL valve 17, such that no direct feedback from the pressure in the patient breathing circuit 7 is provided to the manual input device 18. On the one hand, this is an advantage as control of a correct opening pressure is facilitated. On the other hand, it is still desired to provide a tactile feedback to the user as explained in the introductory part of the specification. In order to further illustrate this need, it is explained that the filling degree of the manual ventilation bladder 13 does not necessarily provide a direct feedback on the pressure in the patient breathing circuit 7.

The operator "feels" the lungs of the patient through the manual ventilation bladder and receives thus a tactile feedback. However, in case the APL valve 17 is adjusted to a low opening pressure, or completely opened, substantially all gas may be vented as excess gas via the APL valve 17. In this case, ventilation of the patient is not ensured, and the manual ventilation bladder 13 becomes empty, i.e. the operator looses any tactile feedback to the patient. Upon such a lack of tactile feedback from the manual ventilation bladder, the user typically adjust the APL valve to a higher opening pressure in order to lower venting of excess gas via the APL valve 17 and in order to raise pressure in the patient breathing circuit. At the same time, the user will flush 02 gas into the patient breathing circuit, for quickly refilling the latter with gas volume.

However, if for instance the APL valve were adjusted to a very high opening pressure, or completely closed, substantially no excess gas may be vented at all and the manual ventilation bladder 13 continues to increase in size. The compliance of a rubber manual ventilation bladder 13 increases with increasing bag size, which means that adding volume to a manual ventilation bladder 13 causes a negligible rise in the pressure until the nominal capacity is reached. When more volume is added at this point, the pressure rises quickly. In order to improve patient safety, the operator of the manual ventilation bladder 13 needs an additional feedback that is provided in another way than by the tactile feedback provided by the filling degree of the manual ventilation bladder 13. This may be provided by the APL valve having increasing operating resistance at higher opening pressure levels, as disclosed in US 5,950,623. According to embodiments described herein, this feedback is provided as a haptic feedback to the user when the user operates a manual input device 18 controlling the opening pressure of the APL valve 17.

Fig. 2 is a schematic illustration of an embodiment of a manual input device 18 for manually adjusting at least one operating parameter of an anesthesia machine, which in the present embodiment is an opening pressure level of the electronic APL valve 17. Adjustment of the opening pressure of the APL valve 17 is made by means of a manual input device 18, which generates an electronic signal in dependence of a mechanical position thereof. The electronic signal is processed and provided to the electronic APL valve 17 for adjustment of the opening pressure thereof. The electronic APL valve 17 itself is located at a position such that it is in pneumatic connection with the patient breathing circuit 7. Depending on the design of the anesthesia machine or the patient breathing circuit, in one mode of operating the patient breathing circuit 7, may be connected to the APL valve 17 only during manual ventilation. This may be provided by a selector knob or mode switch of the anesthesia machine 1, which is set to Bag or Manual ventilation mode. When the selector knob is set to mechanical ventilation mode or controlled ventilation mode, the APL valve may no longer be part of the patient breathing circuit 7. In this mode, even if the APL valve is left open, no gas is able to escape from the patient breathing circuit 7 out of the APL valve 17.

For instance, an electronically adjustable spring compression force controls the opening pressure of the APL valve 17. More precisely, the pressure inside the breathing circuit 7 must generate a force that exceeds the spring compression force for the APL valve to open. Alternatively, other design of an electronic APL valve are provided, such as a pressure regulating valve, similar to known expiratory valves, in order to be able to control the opening pressure of the APL valve in a better way. An alternative electronic APL valve is described in PCT/EP2006/070068.

In any case, as pressure continues to build up from the combination of fresh gas flow and manual compression of the breathing bag, the opening pressure of the APL valve 17 is exceeded and excess gas is vented to a scavenging system.

Furthermore, the manual input device 18 is programmable for a haptic feedback and comprises an operating member 20 for providing the manual input, a detecting unit that detects a movement of the operating member 20, and a haptic feedback unit 22 that applies a mechanical output to the operating member 20 depending on the movement thereof that is detected.

The haptic feedback unit 22 provides an interface of the anesthesia machine that interfaces the operator thereof via the sense of touch by applying mechanical outputs, such as forces, vibrations and/or motions to the operator. In other words, the haptic feedback is based on the operator's physical manipulations of the operating member 20 and provides physical feedback sensations to the operator upon manually manipulating the operating member 20. The actual feedback of the manual input-output device 18 is programmable for instance with regard to a range of motion, damping and stiffness characteristics, spacing, number, and shape of detents, etc. The programmable feedback may also depend itself on other settings of the anesthesia machine 1, such as patient category. The Haptic feedback may be programmable with regard to position, activation force, vigor, intensity, magnitude, direction of operation, etc. of the manual input-output device 18.

In more detail, the embodiment of the haptic feedback input device 18 comprises a manually rotated knob 20. An encoder 23 is arranged to detect the rotational angle of the knob 20. A motor 22 is configured to apply a torque to the knob 20 via shaft 21. A controller unit 25 is configured to control the motor 22 via control signal 27 in response to a rotational angle α detected by the encoder 23, fed via encoder signal 26 to the controller unit 25. The manual rotation of knob 20 is transported to the encoder 23 via the shaft 21, where the rotational angle α is detected. For instance, the knob 20 is rotated clockwise in order to increase the opening pressure of the APL valve 17, and thus the maximum pressure in the patient breathing circuit 7. The controller unit 25 outputs an operating signal 28 in response to the rotational angle α to the electronic APL valve 17, which is a separate apparatus. Depending on the currently adjusted rotational angle α of rotating knob 20, the opening pressure of the electronic APL valve 17 is adjusted accordingly. A torque may be applied to the shaft 21 and thus to knob 20 by the electrical motor 22. The torque of the motor 22 is programmable with regard to intensity and timing. For instance, an increasing torque may be applied to knob 20 from motor 22 in a direction opposite to the rotating direction of the knob 20 with increasing opening pressure selected. This will provide the operator with an increased rotational resistance in the knob, giving an immediate feedback of the range of the opening pressure in which the valve is currently operated.

Instead of obtaining a feeling as the haptic force feedback, the haptic feedback may provide an accelerating feeling by applying a torque to the knob 20 in the same direction as the rotating direction of the knob, or a haptic feedback by which a click feeling is obtained by reversing the torque applied to the knob 20 when the rotational angle of the knob 20 exceeds a predetermined rotational angle.

At start of operation, for instance, when the operation mode of anesthesia machine 1 is switched from mechanical ventilation to manual ventilation and VPL valve 17 as well as input unit 18 are activated, the position of rotatable knob 20 is in an arbitrary rotational start position. However, the position of knob 20 at activation thereof is not of importance. As knob 20 is rotatable without end points in both directions, the current position may be taken as the starting position. Alternatively, fixed, defined positions of knob 20 may be provided, if so desired. In this case, for instance an end position may be defined into which the knob 20 is rotated to when switching to or from manual ventilation mode. This active returning to an initial position may be provided by means of motor 22.

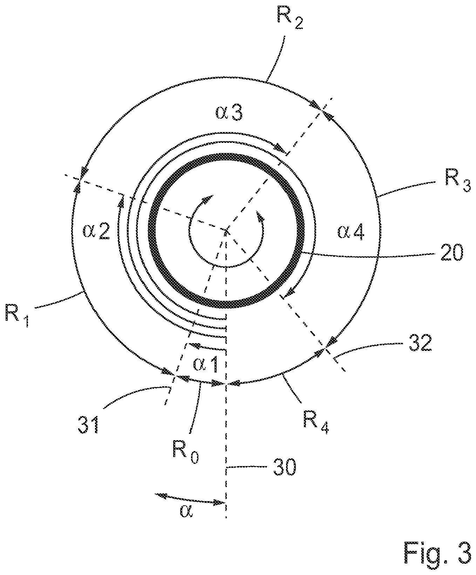

In the present embodiment, the actual end positions sensed by the operator are provided by the haptic feedback provided by motor 22. The encoder 23 may provide relative positions to this initial start position of knob 20 or it may provide absolute angular positions of the knob 20. An initial opening pressure level of APL valve 17 is adjusted by controller unit 25 via signal 28. The initial opening pressure may be programmed to a fix value or adapted to specific user set-ups. For instance, the initial opening pressure may be chosen to be rather low, e.g. in the range of 10 cm H₂O, in order to provide a defined flow into manual ventilation bladder 13, but not to risk any patient injury by too high pressures that may build up in the patient breathing circuit 7. Alternatively, it may be chosen that the opening pressure level may be adjusted at the current position without the need of rotating the knob to a defined position initial opening pressure of the APL valve 17 may be set to 0 cm H₂O, i.e. the APL valve is completely open and the patient breathing circuit 7 is vented. In this case, the knob is initialized to be at rotational position 31, as shown in Fig. 3 and explained further below in more detail. In an embodiment, the initial opening pressure, as well as the entire haptic feedback characteristics of manual input device 18, may be chosen in dependence of a patient category set on anesthesia machine 1. For instance, neonatal patients may be harmed by lower pressures in patient breathing circuit 7 than adult patients. Hence, for neonatal patients the initial opening pressure may be chosen to be very low or zero, whereas adult patients may have an initial opening pressure that is chosen to be much higher.

With reference to Figs. 3, 4A and 4B an embodiment of different operational modes in different rotational ranges of the manual input device 18 are now described in detail.

In the illustrated embodiment, the rotational angle α detected by the encoder 23 is 360 degrees over an entire revolution of knob 20. In other embodiments, a range of the rotational angle α may have another value, larger or smaller than 360 degrees. The entire revolution, as in the embodiment, or alternatively another range of the rotational angle α, may be sub-divided into various adjacent sections or sub-ranges (herein after also called "range", furnished with an index, for the sake of simplicity). In Figs. 3, 4A and 4B ranges R₀, R₁, R₂, R₃, and R₄ are illustrated.

Range R₀ extends from a virtual position of zero degrees at line 30 up to a first rotational angle α₁. Range R₁ extends from the position at line 31 at the first rotational angle α₁ up to a second first rotational angle α₂, Range R₂ extends from the position at the second rotational angle α₂ up to a third rotational angle α₃. Range R₃ extends from the position at the third rotational angle α₃ up to a fourth rotational angle α₄ at line 32. Range R₄ extends from the position at the fourth rotational angle α₄ up to the virtual position of 0 degrees at line 30, completing a entire revolution of knob 20.

In the embodiment line 31 is an end line representing a starting point for adjustment of the opening pressure of the APL valve 17. Hence, range R₀ represents a range where manual input device 18 provides a high resistance counter clockwise, but low resistance clockwise. This is illustrated in Fig. 4A and Fig. 4B. Fig. 4A represent the torque T of motor 22 applied to knob 20 when this is turned by an operator in clockwise direction, as depicted by arrow 41. Fig. 4B illustrates represent the torque T of motor 22 applied to knob 20 when this is turned by an operator in the opposite, counter clockwise, direction, as depicted by arrow 42.

Range R₁ of the illustrated embodiment represents a range of a low opening pressure of the APL valve 17. In range R₁ haptic feedback provided by the torque applied by motor 22 to the axle 21 and knob may be zero, i.e. the knob rotates very easy in both directions only hindered in rotation by a frictional resistance. Alternatively, an increasing torque T may be applied in range R₁ when turning knob 20 in clockwise direction, as depicted by arrow 41. A different torque or even an acceleration may be provided in range R₁ when turning knob 20 in the opposite, counter clockwise, direction, depicted by arrow 42. The operator thus feels that the end point 31 is about to be reached when rotating knob 41 is manually actuated counter clockwise towards the position of first rotational angle α₁.

Range R₂ of the illustrated embodiment represents a range of a mid size opening pressure of the APL valve 17. In range R₂ the haptic feedback provided by the torque T applied by motor 22 to the knob 20 may be increasing in clockwise direction, or as illustrated in Fig. 4A, be on a constant elevated level. However, the knob rotates very easy in the opposite, counter clockwise direction 42. The operator thus feels that mid pressure range R₂ of the opening pressure of APL valve 17 is adjusted.

Range R₃ of the embodiment represents a range of a high opening pressure of the APL valve 17. In range R₃ the haptic feedback provided by the torque T applied by motor 22 to the knob 20 may be further increasing in clockwise direction, towards a maximum torque level "max" or as illustrated in Fig. 4A. However, the knob rotates very easy in the opposite, counter clockwise direction 42. The operator thus feels that high pressure range R₃ of the opening pressure of APL valve 17 is adjusted.

Range R₄ extends from the position at the fourth rotational angle α₄ up to the virtual position of 0 degrees at line 30, completing a entire revolution of knob 20. In Range R₄ the haptic feedback provided by the torque T applied by motor 22 to the knob 20 in clockwise direction is at the maximum torque level "max". However, the knob 20 rotates very easy in the opposite, counter clockwise direction 42.

The transition between ranges R₀, R₁, R₂, R₃, and range R₄ may be fed back to the operator by a detent, providing a "click" feeling to the operator, as illustrated in Figs. 4A and 4B. Such detents given as a haptic feedback may possibly be used for marking predefined pressure levels.

Hence, the haptic feedback is different for each of the ranges R₀, R₁, R₂, R₃, and R₄ and dependant on an operation direction of the knob 20.

Other embodiments, which are not illustrated in the Figs., may have provide a higher resistance counter clockwise than illustrated, which is substantially lower than the resistance in clockwise direction. Also, the haptic profile in the different ranges may be of substantially identical in both directions, but at different levels of resistance, e.g. the "clockwise" curve 40 of Fig. 4A may be displaced parallel to the T-axle forming a "counter-clockwise" curve, corresponding to Fig. 4_{B}, forming a hysteresis like closed haptic feedback profile. This may improve the haptic legibility or distinctness of a current operating range in which the input device is operating.

In embodiments the "clockwise" haptic feedback is significantly different from the "counter" clockwise haptic feedback.

In some embodiments the number of sub-ranges may be different from those described an illustrated with regard to the above-described embodiment. For instance, a single range or a plurality of sub-ranges may have a specific programmed first haptic feedback profile for a certain patient category, and a different, second haptic feedback profile for another patient category. The haptic feedback of some embodiments may also comprise communicating an alarm situation to the user by means of a haptic feedback, like a vibrating or shaking of the input device.

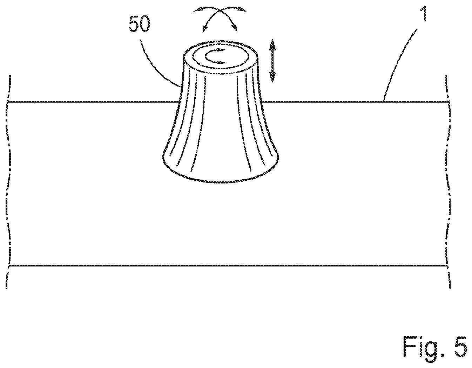

Fig. 5 is a schematic illustration of a further embodiment of manual input device 50 for manually adjusting an opening pressure level of the electronic APL valve 17, as well as other operating parameters of an anesthesia delivery system. The opening pressure of APL valve 17 may be adjusted as described above with reference to Figs. 2-4. The embodiment of manual input device 50 is provided as a multi function rotary knob having several degrees of freedom. For instance the knob may be pressed down in order to activate an O₂ flush. The amount of fresh gas fed into the patient breathing circuit 7 from mixer 3 may be adjusted by tilting the knob in a certain direction. Further operating parameters may be adjusted by tilting the knob in another tilting direction. Haptic feedback may be provided when adjusting any of the operating parameters, e.g. a variable resistance of the knob depending on the fresh gas tilting direction. For instance a haptic feedback may be given when activating or the O₂ flush function, for instance by a click feeling, or a vibrating or tickling up and down movement of the knob. Alternatively, a feedback force directed against the direction of input may be increased with increasing activation time, thus trying to restore the initial knob position.

Alternatively, a separate O₂ flush button may be provided as an embedded or recessed button in the rotary knob. This feature provides increased patient safety, as unintended activation of an O₂ flush is prevented. Safety may further be increased by a haptic feedback indicating the user activation of the O₂ flush.

Moreover, a further function may be integrated into the input device 50. For instance, when lifting the knob upwardly from an initial position, a function such as a pressure release function may be activated. Again, a haptic feedback may be provided upon activation of the pressure release function. A pressure release may be desired to be activated by a user when pressure raises in part of a breathing apparatus that is not covered by other pressure control or pressure limiting valves, like the APL valve. For instance, a locking of a breathing circuit of some breathing apparatuses may occur due to an overpressure, which may result in making a manual ventilation bladder unresilient and even may prohibit compression thereof. This in turn would make it impossible to supply breathing gas to the patient. Therefore, to quickly deflate the gas from the manual ventilation bladder, a pressure release function may be needed. Normally, this pressure release is performed by abruptly disengaging the tubes connecting the breathing apparatus with the patient. By doing so, the gas in the breathing circuit and the manual ventilation bladder is allowed to escape, whereupon the breathing apparatus returns to normal operation. However, when breaking the breathing circuit by disengaging the patient tubes, breathing gas, perhaps containing anesthetic agents, is released therefrom and operating personal is exposed to the breathing gas.

When pressure is released, upon lifting the knob, a pressure release from the part of the breathing apparatus in question is actuated by a pressure release unit of the breathing apparatus in a suitable way. The knob may by itself return to the initial position when released, e.g. by a suitable spring force, or by using the haptic feedback unit. The undesired exposure of operating personnel to breathing gas perhaps containing anesthetic agents, is avoided thanks to the pressure release function, as released gas may be evacuated in a controlled way from the breathing apparatus by a suitable pressure release unit, e.g. a pressure release valve fluidly connected to an evacuation line.

By gathering multiple functionalities in input device 50, multiple functions may be performed from a single input device, allowing simple handgrips. This may result in improved functionality, as the operator does not need to disengage patient tubes, and thus workload decreases, as well as a less stressful situation for the operator occurs, and hence patient safety is increased. Furthermore, input device 50 may save valuable space on the breathing apparatus.

Alternatively, a pressure release functionality may be controlled by a separate input device, that may be provided with haptic feedback.

In the present context an APL valve is only used during a manual or spontaneous ventilation mode of the anesthesia machine 1. However, the APL valve may also be operated in modes where it remains in pneumatic connection with the patient breathing circuit even when the selector knob is set to a mechanical ventilation mode. Even in this case the manual input device may provide a haptic feedback with regard to the adjusted opening pressure, if so desired.

The present invention has been described above with reference to specific embodiments. However, other embodiments than the above described are equally possible within the scope of the invention. Different method steps than those described above, performing the method by hardware or software, may be provided within the scope of the invention as defined by the appended patent claims. The different features and steps of embodiments of the invention may be combined in other combinations than those described above.

As used herein, the singular forms "a", "an" and "the" are intended to include the plural forms as well, unless expressly stated otherwise. It will be further understood that the terms "includes," "comprises," "including" and/or "comprising," when used in this specification, specify the presence of stated features, integers, steps, operations, elements, and/or components, but do not preclude the presence or addition of one or more other features, integers, steps, operations, elements, components, and/or groups thereof. It will be understood that when an element is referred to as being "connected" or "coupled" to another element, it can be directly connected or coupled to the other element or intervening elements may be present. Furthermore, "connected" or "coupled" as used herein may include wirelessly connected or coupled. As used herein, the term "and/or" includes any and all combinations of one or more of the associated listed items.

Unless otherwise defined, all terms (including technical and scientific terms) used herein have the same meaning as commonly understood by one of ordinary skill in the art to which this invention belongs. It will be further understood that terms, such as those defined in commonly used dictionaries, should be interpreted as having a meaning that is consistent with their meaning in the context of the relevant art and will not be interpreted in an idealized or overly formal sense unless expressly so defined herein.

## Claims

1. A breathing apparatus (1) comprising a manual input-output device (18), wherein said manual input-output device (18) is devised for providing a manual input for adjusting at least one patient critical operating parameter of said breathing apparatus (1), wherein said manual input-output device (18) is programmable for a haptic feedback upon adjustment of said at least one patient critical operating parameter by said manual input-output device (18), and comprises
an operating member (20) that is adapted to provide said manual input of said at least one patient critical operating parameter, and that is adapted to provide a manual output,
a detecting unit that is configured to detect a movement of said operating member (20) upon said manual input for adjusting at least one patient critical operating parameter of said breathing apparatus (1), and **characterized by**
a haptic feedback unit (22) that is configured to apply said manual output as a mechanical output to said operating member (20) depending on said movement detected.

2. The breathing apparatus (1) according to claim 1, whereby a resistance of said operating member (20) at a point of operation thereof is different in opposite operating directions.

3. The breathing apparatus (1) according to claim 1 or 2, wherein said operating member (20) is a rotatable knob, and wherein said mechanical output is a torque applied to said rotatable knob when operated.

4. The breathing apparatus (1) according to any preceding claim, wherein said detecting unit is an electromagnetic encoder and said haptic feedback unit (22) is an electrical motor.

5. The breathing apparatus (1) according to any preceding claim, wherein said haptic feedback unit (22) is configured to provide information to said operator concerning a range of a current value of said at least one patient critical operating parameter by means of said mechanical output to said operating member (20).

6. The breathing apparatus (1) according to claim 5, wherein said haptic feedback unit (22) is configured to provide a specific haptic feedback when passing between different ranges.

7. The breathing apparatus (1) according to any preceding claim, wherein said patient critical operating parameter is an opening pressure of an adjustable pressure-limiting valve (17) in an anesthesia breathing circuit (7) of said breathing apparatus (1).

8. The breathing apparatus (1) according to any of claims 1-6, wherein said patient critical operating parameter is comprised in the list of positive end-expiratory pressure (PEEP), upper inspiratory pressure limit, tidal volume, pop off pressure, freshgas flow, gas concentration, such as oxygen (O₂) concentration, nitrous oxide (N₂O) concentration, nitric oxide (NO) concentration, or concentration of an anesthetic agent.

9. The breathing apparatus (1) according to any preceding claim, wherein said manual input-output device in addition is devised to be operated without haptic feedback for parameters other than said patient critical operating parameters.

10. The breathing apparatus (1) according to any preceding claim, wherein said manual input-output device is programmable for said haptic feedback by providing individual haptic feedback profiles for each of the patient critical operating parameters, such as with regard to position, activation force, vigor, intensity, magnitude, or direction of operation thereof.

11. The breathing apparatus (1)) according to any preceding claim, wherein said programmable haptic feedback is adjustable dependent on different patient categories.

12. The breathing apparatus (1) according to any preceding claim, wherein said manual input-output device (18) is provided a as a multi function rotary knob having several degrees of freedom, each degree of freedom devised to adjust at least one of a plurality of said patient critical operating parameters, and devised to give said haptic feedback in at least one degree of freedom of said several degrees of freedom.

## Patentansprüche

1. Atemgerät (1) umfassend eine manuelle Eingabe-Ausgabevorrichtung (18), wobei die manuelle Eingabe-Ausgabevorrichtung (18) für das Bereitstellen einer manuellen Eingabe für das Justieren von mindestens einem für einen Patienten lebensnotwendigen Betriebsparameter des Atemgeräts (1) ausgelegt ist, wobei die manuelle Eingabe-Ausgabevorrichtung (18) für eine haptische Rückmeldung nach der Justierung des mindestens einen für einen Patienten lebensnotwendigen Betriebsparameters durch die manuelle Eingabe-Ausgabevorrichtung (18) programmierbar ist, und umfasst
ein Betriebsorgan (20), das für das Bereitstellen der manuellen Eingabe des mindestens einen für einen Patienten lebensnotwendigen Betriebsparameters angepasst ist, und das für das Bereitstellen einer manuellen Ausgabe angepasst ist,
eine Erfassungseinheit, die für das Erfassen einer Bewegung des Betriebsorgans (20) nach der manuellen Eingabe zum Justieren mindestens eines für einen Patienten lebensnotwendigen Betriebsparameters des Atemgeräts (1) ausgelegt ist, und **gekennzeichnet durch**
eine haptische Rückmeldungseinheit (22), die zum Anwenden der manuellen Ausgabe als eine mechanische Ausgabe auf dem Betriebsorgan (20) in Abhängigkeit von der erfassten Bewegung ausgelegt ist.

2. Atemgerät (1) nach Anspruch 1, wobei ein Widerstand des Betriebsorgans (20) zu einem Betriebspunkt davon in entgegengesetzten Betriebsrichtungen unterschiedlich ist.

3. Atemgerät (1) nach Anspruch 1 oder 2, wobei das Betriebsorgan (20) ein Drehknopf ist, und wobei die mechanische Ausgabe ein Drehmoment ist, das während des Betriebs auf den Drehknopf ausgeübt wird.

4. Atemgerät (1) nach einem der vorgehenden Ansprüche, wobei die Erfassungseinheit ein elektromagnetischer Kodierer ist und die haptische Rückmeldungseinheit (22) ein elektrischer Motor ist.

5. Atemgerät (1) nach einem der vorgehenden Ansprüche, wobei die haptische Rückmeldungseinheit (22) dazu ausgelegt ist, dem Betreiber Informationen betreffend einen Bereich eines aktuellen Werts des mindestens einen für einen Patienten lebensnotwendigen Betriebsparameters mittels der mechanischen Ausgabe an das Betriebsorgan (20) bereitzustellen.

6. Atemgerät (1) nach Anspruch 5, wobei die haptische Rückmeldungseinheit (22) dazu ausgelegt ist, eine spezifische haptische Rückmeldung bereitzustellen, wenn sie zwischen verschiedenen Bereichen passiert.

7. Atemgerät (1) nach einem der vorgehenden Ansprüche, wobei der für einen Patienten lebensnotwendige Betriebsparameter ein Öffnungsdruck eines justierbaren druckbegrenzenden Ventils (17) in einem Anästhesie-Atemkreislauf (7) des Atemgeräts (1) ist.

8. Atemgerät (1) nach einem der Ansprüche 1-6, wobei der für einen Patienten lebensnotwendige Betriebsparameter in der folgenden Liste enthalten ist: positiver endexpiratorischer Druck (PEEP), Obergrenze des inspiratorischen Drucks, Tidalvolumen, Pop-Off-Druck, Frischgasstrom, Gaskonzentration, wie beispielsweise Sauerstoff (O₂)-Konzentration, Distickstoffoxid (N₂O)-Konzentration, Stickoxid (NO)-Konzentration, oder Konzentration eines Anästhesiemittels.

9. Atemgerät (1) nach einem der vorgehenden Ansprüche, wobei die manuelle Eingabe-Ausgabevorrichtung ferner dazu ausgelegt ist, ohne haptische Rückmeldung für andere Parameter als die für einen Patienten lebensnotwendigen Betriebsparameter betrieben zu werden.

10. Atemgerät (1) nach einem der vorgehenden Ansprüche, wobei die manuelle Eingabe-Ausgabevorrichtung für die haptische Rückmeldung durch Bereitstellen individueller haptischen Rückmeldungsprofile für jeden der für einen Patienten lebensnotwendigen Betriebsparameter, wie beispielsweise in Bezug auf Position, Betätigungskraft, Vitalität, Intensität, Magnitude oder Betriebsrichtung davon, programmierbar ist.

11. Atemgerät (1) nach einem der vorgehenden Ansprüche, wobei die programmierbare haptische Rückmeldung in Abhängigkeit von verschiedenen Patientenkategorien justierbar ist.

12. Atemgerät (1) nach einem der vorgehenden Ansprüche, wobei die manuelle Eingabe-Ausgabevorrichtung (18) als ein multifunktioneller Drehknopf vorgesehen ist, der mehrere Freiheitsgrade aufweist, wobei jeder Freiheitsgrad dazu ausgelegt ist, mindestens einen aus einer Mehrzahl der für einen Patienten lebensnotwendigen Betriebsparameter zu justieren, und dazu ausgelegt ist, die haptische Rückmeldung in mindestens einem Freiheitsgrad der mehreren Freiheitsgrade zu geben.

## Revendications

1. Appareil respiratoire (1), comprenant un dispositif manuel d'entrée-sortie (18), ledit dispositif manuel d'entrée-sortie (18) étant conçu pour fournir une entrée manuelle pour régler au moins un paramètre critique de fonctionnement d'un patient dudit appareil respiratoire (1), ledit dispositif manuel d'entrée-sortie (18) étant programmable pour une rétroaction haptique après le réglage dudit au moins un paramètre de fonctionnement critique du patient par ledit dispositif manuel d'entrée-sortie (18), et comprend
un élément de fonctionnement (20) adapté pour fournir ladite entrée manuelle dudit au moins un paramètre de fonctionnement critique du patient, et qui est adapté pour fournir une sortie manuelle,
une unité de détection qui est configurée pour détecter un mouvement dudit élément de fonctionnement (20) après ladite entrée manuelle pour régler au moins un paramètre critique de fonctionnement du patient dudit appareil respiratoire (1), et **caractérisé par**
une unité de rétroaction haptique (22) qui est configurée pour appliquer ladite sortie manuelle en tant que sortie mécanique audit élément de fonctionnement (20) en fonction dudit mouvement détecté.

2. Appareil respiratoire (1) selon la revendication 1, dans lequel une résistance dudit élément de fonctionnement (20) à un point de fonctionnement de celui-ci est différente dans des directions de fonctionnement opposées.

3. Appareil respiratoire (1) selon la revendication 1 ou 2, dans lequel ledit élément de fonctionnement (20) est un bouton rotatif, et dans lequel ladite sortie mécanique est un couple appliqué audit bouton rotatif lorsqu'il est actionné.

4. Appareil respiratoire (1) selon l'une quelconque des revendications précédentes, dans lequel ladite unité de détection est un codeur électromagnétique, et ladite unité de rétroaction haptique (22) est un moteur électrique.

5. Appareil respiratoire (1) selon l'une quelconque des revendications précédentes, dans lequel ladite unité de rétroaction haptique (22) est configurée pour fournir des informations audit opérateur concernant une plage d'une valeur actuelle dudit au moins un paramètre de fonctionnement critique du patient au moyen de ladite sortie mécanique vers ledit élément de fonctionnement (20).

6. Appareil respiratoire (1) selon la revendication 5, dans lequel ladite unité de rétroaction haptique (22) est configurée pour fournir une rétroaction haptique spécifique lors du passage entre différentes plages.

7. Appareil respiratoire (1) selon l'une quelconque des revendications précédentes, dans lequel ledit paramètre critique de fonctionnement du patient est une pression d'ouverture d'une soupape de limitation de pression réglable (17) dans un circuit respiratoire anesthésique (7) dudit appareil respiratoire (1).

8. Appareil respiratoire (1) selon l'une quelconque des revendications 1 à 6, dans lequel ledit paramètre de fonctionnement critique du patient est compris dans la liste de la pression expiratoire positive (PEEP), de la pression inspiratoire supérieure, du volume courant, de la surpression, du débit de gaz frais, de la concentration de gaz, telle que la concentration en oxygène (O₂), la concentration en oxyde nitreux (N₂O), la concentration en oxyde nitrique (NO) ou la concentration d'un agent anesthésique.

9. Appareil respiratoire (1) selon l'une quelconque des revendications précédentes, dans lequel ledit dispositif d'entrée-sortie manuel est en outre conçu pour fonctionner sans rétroaction haptique pour des paramètres autres que lesdits paramètres de fonctionnement critiques du patient.

10. Appareil respiratoire (1)) selon l'une quelconque des revendications précédentes, dans lequel ledit dispositif manuel d'entrée-sortie est programmable pour ladite rétroaction haptique en fournissant des profils de rétroaction haptique individuels pour chacun des paramètres de fonctionnement critiques du patient, tels que position, force d'activation, vigueur, intensité, amplitude ou direction de fonctionnement de celui-ci.

11. Appareil respiratoire (1) selon l'une quelconque des revendications précédentes, dans lequel ladite rétroaction haptique programmable est réglable en fonction de différentes catégories de patients.

12. Appareil respiratoire (1) selon l'une quelconque des revendications précédentes, dans lequel ledit dispositif manuel d'entrée-sortie (18) est pourvu en tant que bouton rotatif multifonctionnel ayant plusieurs degrés de liberté, chaque degré de liberté étant conçu pour régler au moins l'un d'une pluralité desdits paramètres de fonctionnement critiques du patient, et conçu pour donner ladite rétroaction haptique dans au moins un degré de liberté desdits plusieurs degrés de liberté.
